# EUROPÄISCHE PATENTSCHRIFT

(11) **EP 1 437 356 B1**
(45) Veröffentlichungstag und Bekanntmachung des Hinweises auf die Patenterteilung: **29.03.2006**
(21) Anmeldenummer: 03029975.4
(22) Anmeldetag: 30.12.2003
(51) Int. Cl.: C07F 1/08

(54) **Kupferkomplexe und ihre Verwendung**
Copper complexes and the use thereof
Cu complexes et leur utilisation

(30) Priorität: 07.01.2003 DE 10300097
(43) Veröffentlichungstag der Anmeldung: 14.07.2004
(73) Patentinhaber: LANXESS Deutschland GmbH, 51369 Leverkusen (DE)
(72) Erfinder: Scholz, Ulrich, Dr., 45475 Mülheim (DE); Kunz, Klaus, Dr., 40235 Düsseldorf (DE); Gaertzen, Oliver, Dr., 50668 Köln (DE); Benet-Buchholz, Jordi, Dr., 51375 Leverkusen (DE); Wesener, Joachim, Dr., 51061 Köln (DE)

(56) Entgegenhaltungen:
- EP-A- 0 679 438
- DE-A1- 2 431 330
- US-A- 4 319 050
- DOYLE, MICHAEL P. ET AL: "Influence of olefin coordination of cyclopropanation selectivity" TETRAHEDRON LETTERS , 25(37), 4087-90 CODEN: TELEAY; ISSN: 0040-4039, 1984, XP002277654
- DATABASE CHEMABS [Online] CHEMICAL ABSTRACTS SERVICE, COLUMBUS, OHIO, US; GRAHAM, PETER M. ET AL: "Coordination Polymers of Copper(I) Halides" XP002277655 gefunden im STN Database accession no. 2000:713469 & INORGANIC CHEMISTRY , 39(22), 5121-5132 CODEN: INOCAJ; ISSN: 0020-1669, 2000,

## Beschreibung

Die Erfindung betrifft Kupferkomplexe von Phosphorverbindungen, ein Verfahren zu deren Herstellung sowie ihre Verwendung in katalytischen Kupplungsreaktionen.

Die von Kupferkomplexen katalysierte Verknüpfung von Arylhalogeniden oder -sulfonaten mit Heteroatomfunktionalitäten wie beispielweise Thiolen, Aminen und Amiden ist in der Literatur häufig beschrieben worden.

Als Liganden dienen dabei häufig Stickstoff- oder Phosphorverbindungen. So ist beispielsweise aus Venkataraman et al. (Tetrahedron Letters, 2001, 42, 4791-4793) bekannt, präformierte Komplexe aus Kupferdibromid und Triphenylphosphin zur Addition von Arylhalogeniden an sekundäre aromatische Amine einzusetzen. Nachteilig an den bislang bekannten Kupferkomplexen von Phosphorverbindungen sind jedoch die oftmals nur geringe Chemoselektivität und das schmale Spektrum an Reaktionen, in denen technisch akzeptable Umsätze und Umsatzraten erzielt werden können.

DE-A 24 31 330 beschreibt ein Verfahren zur Herstellung von Kohlensäureestern aus CO und Sauerstoff durch Umsetzung eines aliphatischen oder cycloaliphatischen Alkohols in Gegenwart von Kupferkomplexen als Katalysatoren. Die Handhabung von CO und Sauerstoff machen das Verfahren großtechnisch problematisch, zudem wird keine Umsetzung aromatischer Alkohole beschrieben.

Es bestand daher das Bedürfnis Kupferkomplexe von Phosphorverbindungen bereitzustellen, die einfach herzustellen sind und in Kupplungsreaktionen in guten Ausbeuten die gewünschten Produkte liefern.

Gegenstand der vorliegenden Erfindung ist daher ein Verfahren zu Herstellung von Verbindungen der Formel (IV),

Ar-(F-R²)ₙ (IV)

in der
- n: für 1, 2 oder 3 steht und
- Ar: für einen substituierten oder unsubstituierten aromatischen Rest und
- F: für Sauerstoff, Schwefel, NR³, NR³CO oder Ethindiyl steht, wobei R³ für Wasserstoff, C₁-C₁₂-Alkyl, C₅-C₁₈-Aryl oder C₆-C₁₉-Arylalkyl steht und
- R²: für Ar, C₁-C₁₂-Alkyl, C₁-C₁₂-Halogenalkyl, C₂-C₁₂-Alkenyl oder C₆-C₁₉-Arylalkyl steht,
das dadurch gekennzeichnet ist, dass Verbindungen der Formel (V)

Ar-Z (V)

in der
- Ar: vorstehend genannte Bedeutung besitzt und
- Z: für Chlor, Brom, Iod, ein Diazoniumsalz oder ein Sulfonat steht
mit Verbindungen der Formel (VI) umgesetzt werden,

H-F-R² (VI)

in der F und R² die vorstehend genannte Bedeutung besitzen und
wobei die Umsetzung in Gegenwart von Base und Verbindungen der Formel (I) erfolgt, 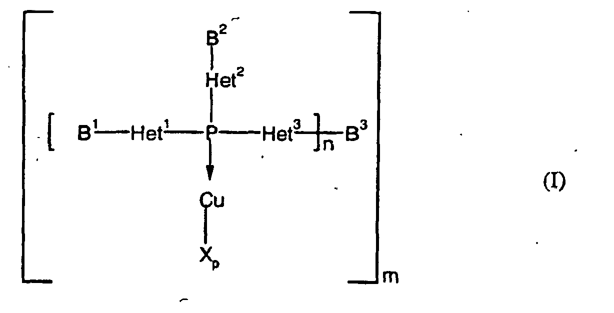 in der
- Het¹, Het² und Het³: jeweils unabhängig voneinander fehlen, für Sauerstoff oder NR¹ stehen, wobei R¹ für C₁-C₈-Alkyl, C₅-C₁₈-Aryloder C₆-C₁₉-Arylalkyl steht und
- B¹ und B²: jeweils unabhängig voneinander für C₁-C₈-Alkyl, C₅-C₁₈-Aryl oder C₆-C₁₉-Arylalkyl stehen oder wobei die Reste B¹ und B² gemeinsam für einen divalenten Rest mit insgesamt 2 bis 40 Kohlenstoffatomen stehen können und
- B³: für C₁-C₈-Alkyl, C₅-C₁₈-Aryl, C₆-C₁₉-Arylalkyl oder einen Rest mit insgesamt 2 bis 40 Kohlenstoffatomen und der Wertigkeit n steht,
- X: für Halogenid, (C₁-C₈-Halogenalkyl)carboxylat, (C₁-C₈-Alkyl)carboxylat, (C₁-C₈-Halogenalkyl)sulfonat, (C₅-C₁₈-Aryl)sulfonat, Cyanid, gegebenenfalls fluoriertes Acetylacetonat, Nitrat, Oxinat, Phosphat, Carbonat, Hexafluorophosphat, Tetraphenylborat, Tetrakis(pentafluorphenyl)borat oder Tetrafluoroborat steht, und
- p: für 0, 1 oder 2 steht und
- n: für 1, 2 oder 3 steht und
- m: für 1, 2 , 3, 4, 5 oder 6 steht.

Im Rahmen der Erfindung können alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Weise kombiniert werden.

**Alkyl** beziehungsweise **Alkoxy** beziehungsweise **Alkylen** beziehungsweise **Alkenylen** bedeutet jeweils unabhängig einen geradkettigen, cyclischen, verzweigten oder unverzweigten Alkylbeziehungsweise Alkoxy- beziehungsweise Alkylen beziehungsweise Alkenylen-Rest, der gegebenenfalls weiter durch C₁-C₄-Alkoxy weiter substituiert sein kann. Gleiches gilt für den nichtaromatischen Teil eines Arylalkyl-Restes.

C₁-C₄-Alkyl steht beispielsweise für Methyl, Ethyl, n-Propyl, iso-Propyl, n-Butyl, sec.-Butyl und tert.-Butyl, C₁-C₈-Alkyl darüber hinaus beispielsweise für n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, 1-Ethyl-2-methylpropyl, n-Heptyl und n-Octyl, C₁-C₁₂-Alkyl weiter darüber hinaus beispielsweise für Adamantyl, die isomeren Menthyle, n-Nonyl, n-Decyl und n-Dodecyl C₁-C₁₈-Alkyl noch weiter darüber hinaus beispielsweise für n-Octadecyl.

C₁-C₄-Alkoxy steht beispielsweise für Methoxy, Ethoxy, n-Propoxy, iso-Propoxy, n-Butoxy, sec.-Butoxy und tert.-Butoxy, C₁C₈-Alkoxy darüber hinaus für n-Pentoxy, 1-Methylbutoxy, 2-Methylbutoxy, 3-Methylbutoxy, neo-Pentoxy, 1-Ethylpropoxy, cyclo-Hexoxy, cyclo-Pentoxy, n-Hexoxy und n-Octoxy, C₁-C₁₂-Alkoxy weiter darüber hinaus beispielsweise für Adamantoxy, die isomeren Menthoxy-Reste, n-Decoxy und n-Dodecoxy.

C₁-C₈-Alkylen steht beispielsweise für Methylen, 1,1-Ethylen, 1,2-Ethylen, 1,1-Propylen, 1,3-Propylen, 1,4-Butylen, 1,2-cyclo-Hexoxylen und 1,2-cyclo-Pentylen.

Halogenalkyl bedeutet jeweils unabhängig einen geradkettigen, cyclischen, verzweigten oder unverzweigten Alkyl-Rest, der einfach, mehrfach oder vollständig durch Chlor- oder Fluoratome substituiert ist.

Beispielsweise steht C₁-C₈-Halogenalkyl für Trifluormethyl, Chlordifluormethyl, 2,2,2-Trifluorethyl, Pentafluorethyl, Nonafluorbutyl, Heptafluorisopropyl, und Perfluoroctyl.

Aryl steht jeweils unabhängig für einen heteroaromatischen Rest mit 5 bis 18 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, oder und vorzugsweise jedoch für einen carbocyclischen aromatischen Rest mit 6 bis 18 Gerüstkohlenstoffatomen.

Beispiele für carbocyclische aromatische Reste mit 6 bis 18 Gerüstkohlenstoffatomen sind zum Beispiel Phenyl, Naphtyl, Phenanthrenyl, Anthracenyl oder Fluorenyl, heteroaromatische Reste mit 5 bis 14 Gerüstkohlenstoffatomen in denen keines, ein, zwei oder drei Gerüstkohlenstoffatome pro Zyklus, im gesamten Molekül mindestens jedoch ein Gerüstkohlenstoffatom, durch Heteroatome, ausgewählt aus der Gruppe Stickstoff, Schwefel oder Sauerstoff, substituiert sein können, sind beispielsweise Pyridinyl, Oxazolyl, Benzofuranyl, Dibenzofuran-yl oder Chinolinyl.

Weiterhin kann der carbocyclische aromatische Rest oder heteroaromatische Rest mit bis zu fünf gleichen oder verschiedenen Substituenten pro Cyclus substituiert sein, die ausgewählt sind aus der Gruppe freies oder geschütztes Hydroxy, Cyano, Chlor, Fluor, C₁-C₁₂-Alkyl, CO(C₁-C₁₂₋Alkyl), COO(C₁-C₁₂-Alkyl), CO(C₅-C₁₈-Aryl), COO(C₅-C₁₈-Aryl), CON(C₁-C₁₂-Alkyl)_{2'} C₅-C₁₈₋Aryl, C₁-C₁₂-Halogenalkyl, C₁-C₁₂-Alkoxy, C₁-C₁₂-Halogenalkoxy, Di(C₁-C₈-alkyl)amino oder Tri(C₁-C₆-alkyl)siloxyl.

Arylalkyl bedeutet jeweils unabhängig einen geradkettigen, cyclischen, verzweigten oder unverzweigten Alkyl-Rest nach vorstehender Definition, der einfach, mehrfach oder vollständig durch Aryl-Reste gemäß vorstehender Definition substituiert sein kann.

Im Folgenden werden die bevorzugten Substitutionsmuster definiert:
- Het¹, Het² und Het³: stehen bevorzugt jeweils unabhängig voneinander für Sauerstoff oder fehlen,
- B¹ und B²: stehen bevorzugt jeweils unabhängig voneinander für sekundäres oder tertiäres C₃-C₈- bzw. C₄-C₈-Alkyl, C₅-C₁₈-Aryl oder Bis-(C₅-C₁₈-Aryl. Ebenfalls bevorzugt stehen B¹ und B² gemeinsam für einen divalenten Rest der ausgewählt ist aus der Gruppe 1,2-Phenylen, 1,3-Phenylen, 1,2-Cyclohexyl, 1,1'-Ferrocenyl, 1,2-Ferrocenyl, 2,2'-(1,1'-Binaphtyl) und 1,1'-Biphenyl, wobei die genannten Reste gegebenenfalls einfach oder mehrfach durch Cyano, Chlor, Fluor, C₁-C₁₂-Alkyl, C₁-C₁₂-Halogenalkyl, C₁-C₁₂-Alkoxy, C₁-C₁₂-Halogenalkoxy, Di(C₁-C₈-alkyl)amino oder Tri(C₁-C₈-alkyl)siloxyl substituiert sein können,
- B³: steht bevorzugt für sekundäres oder tertiäres C₃-C₈- bzw. C₄-C₈-Alkyl, C₅-C₁₈-Aryl, C₆-C₁₉-Arylalkyl oder einen Rest mit insgesamt 2 bis 40 Kohlenstoffatomen und der Wertigkeit n,
- X: steht bevorzugt für Chlorid, Bromid oder Iodid, Trifluormethansulfonat, Trifluoracetat, Methansulfonat, Benzolsulfonat, Cyanid, gegebenenfalls fluoriertes Acetylacetonat, Hexafluorophosphat oder Tetrafluoroborat, wobei Chlorid, Bromid oder Iodid besonders bevorzugt sind.
- n: steht bevorzugt für 1 oder 2,
- m: steht bevorzugt für 1 oder 2.

Es hat sich gezeigt, dass sich ein Verhältnis von Kupfer zu Phosphoratomen im Komplex, wie durch Formel (I) dargestellt, insbesondere dann sehr einfach oder automatisch einstellt, wenn der Ligand zumindest ein Poly(C₅-C₁₈)aryl- oder (C₉-C₁₈)aryl-Strukturelement oder als Strukturelement zumindest ein in ortho-Stellung durch sekundäres oder tertiäres Alkyl substituiertes (C₅-C₁₈)Aryl- Strukturelement enthält oder bei Liganden, die mehr als ein Phosphoratom enthalten B³ nicht für 1,1'-Bisarylen, 2,2'-Bisarylen, 1,2-Aryten oder 1,2-, 1,3- oder 1,4-(C₁-C₈)-Alkylen steht..

Die Formel (I) beinhaltet insbesondere Kupfer-Phosphin-Komplexe, Kupfer-Phosphonit-Komplexe und Kupfer-Phosphit-Komplexe.

Bevorzugte Kupfer-Phosphin-Komplexe, die zudem Gegenstand der vorliegenden Erfindung sind, sind beispielsweise solche, die als Phosphin-Liganden folgende enthalten:
Bis(2-dicyclohexylphosphino)-2'-(N,N-dimethyl-amino)-biphenyl, 2-(Dicyclohexylphosphino)biphenyl, 2-(Dicyclohexylphosphino)-2'-methylbiphenyl, 2-(Di-tert.-butylphosphino)biphenyl oder 2-(Bisdiphenylphosphino)binaphthyl.
Bevorzugt sind 2-(Di-tert.-butylphosphino)biphenyl oder 2-(Dicyclohexylphosphino)biphenyl.
Besonders bevorzugte Kupfer-Phosphin-Komplexe sind [(µ-Br)₂(2-(di-tertiär-butylphosphino)biphenyl}₂Cu₂], [(µ-Trifluormethansulfonato)₂{2-(di-tertiär-butylphosphino)biphenyl}₂Cu₂], [(µ-Br)₂{2-(dicyclohexylphosphino)biphenyl}₂Cu₂] und [(µ-Trifluormethansulfonato)₂{2-(dicyclohexylphosphino)biphenyl}₂Cu₂].
Bevorzugte Kupfer-Phosphonit-Komplexe, die zudem Gegenstand der vorliegenden Erfindung sind, sind beispielsweise solche, die als Phosphonit-Liganden folgende enthalten:
1,1'-Biphenyl-2-yl-dialkylphosphonite wie beispielsweise und bevorzugt 1,1'-Biphenyl-2-yl-dicyclohexylphosphonit und 1,1'-Biphenyl-2-yl-ditert.-butylphosphonit, 3-[(diisopropylphosphino)oxy]phenyldiisopropylphosphonit, 3-[(Di-tert.-butyl-phosphino)oxy]phenyl-di-tert.-butylphosphonit, 3-[(Diphenylphosphino)oxy]phenyl-diphenylphosphonit oder 3-[(dicyclohexylphosphino)oxy]phenyl-dicyclohexylphosphonit, wobei 3-[(diisopropylphosphino)oxy]phenyl-diisopropylphosphonit noch weiter bevorzugt ist.

Ein besonders bevorzugter Kupfer-Phosphonit-Komplex ist [(µ-Br)₂{2-(di-tertiär-butylphosphino)biphenyl)₂Cu₂].

Bevorzugte Kupfer-Phosphit-Komplexe, die zudem Gegenstand der vorliegenden Erfindung sind, sind beispielsweise solche, die als Phosphit-Liganden folgende enthalten:
1,1'-Binaphthyl-2,2'-diyl-iso-propyl-phosphit, 2,4,8,10-Tetra-tert.-butyl-6-phenoxy-12H-di-benzo[d,g][1,3,2]dioxaphosphocin, wobei 1,1'-Binaphthyl-2,2'-diyl-iso-propyl-phosphit und Tris(2,4-di-tertiär-butylphenyl)phosphit bevorzugt sind.

Ein besonders bevorzugter Kupfer-Phosphit-Komplex ist [(µ-Br)₂(1,1'-Binaphthyl-2,2'-diyl-iso-propyl-phosphit₂}Cu₂].

Die Kupfer-Phosphor-Komplexe der Formel (I) können beispielsweise in an sich bekannter Weise durch Umsetzung von Phosphor-Verbindungen der Formel (II) 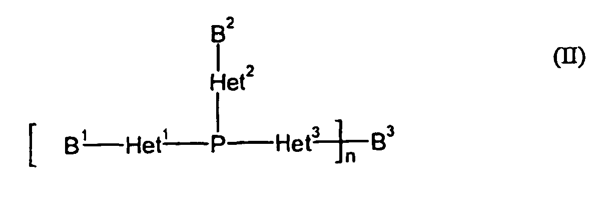 in der n, B¹, B², B³, Het¹, Het² und Het³ die unter der Formel (I) genannten Bedeutungen und Vorzugsbereiche besitzen mit Verbindungen der Formel (III)

Cu-Xₚ (III)

in der X und p die in der Formel (I) genannten Bedeutungen und Vorzugsbereiche besitzen, hergestellt werden.

In Fällen in denen p = 0 ist kann beispielsweise auch Kupferpulver eingesetzt werden. Bevorzugte Verbindungen der Formel (III) sind:
Kupfer(I)oxid, Kupfer(II)oxid, Kupfer(I)-chlorid, Kupfer(I)-trifluormethansulfonat, Kupfer(I)-bromid, Kupfer(I)iodid, Kupfer(II)-bromid, Kupfer(II)-chlorid, Kupfer(II)-acetat, Kupfer(II)-acetylacetonat oder Mischungen davon.

Das molare Verhältnis von Phosphoratomen in Verbindungen der Formeln (II) zu Kupferatomen in Verbindungen der Formel (III) kann bei der Herstellung von komplexen der Formel (I) im Allgemeinen 10:1 bis 0,5:1, bevorzugt 2:1 bis 1:1, besonders bevorzugt 1,2:1 bis 1:1 betragen.

Die Verbindungen der Formel (I) können separat in einem dafür geeigneten inerten organischen Lösungsmittel wie beispielsweise Tetrahydrofuran, Diethylether, Toluol, Xylol, Chloroform, Dichlormethan, Methanol und/oder Ethanol hergestellt werden.

Die günstigste Menge an einzusetzendes Lösungsmittel kann durch entsprechende Vorversuche bestimmt werden.

Die Herstellung der Verbindungen der Formel (I) aus den beschriebenen Ausgangsverbindungen der Formeln (II) und (III) erfolgt dann beispielsweise durch einfaches Zusammengeben der beiden Ausgangsverbindungen in Lösung bei Raumtemperatur.

Es ist auch möglich, die Verbindungen der Formel (I) in-situ in einer Katalysemischung herzustellen. Dazu kann der Einsatz der Verbindungen der Formel (II) auch in Form von Phosphoniumsalzen wie zum Beispiel Tetrafluoroboraten erfolgen (siehe auch Netherton, M.R.; Fu, G.C.; Organic Letters (2001), 3(26), 4295-429).

Die erfindungsgemäßen Verbindungen der Formel (I) eignen sich insbesondere zur Knüpfung von Kohlenstoff-Stickstoff-, Kohlenstoff-Sauerstoff und Kohlenstoff-Schwefelbindungen sowie zur Herstellung von Arylalkinen.

Daher sind von der Erfindung auch Katalysatoren umfasst, die die erfindungsgemäßen Verbindungen der Formel (I) enthalten.

Im Folgenden werden die Vorzugsbereiche für Verbindungen der Formeln (IV) bis (VI) definiert:
- Ar: steht bevorzugt für carbocyclische aromatische Reste mit 6 bis 24 Gerüstkohlenstoffatomen oder für heteroaromatische Reste mit 5 bis 24 Gerüstatomen, in denen keines, ein, zwei oder drei Gerüstatome pro Cyclus, im gesamten Molekül mindestens jedoch ein Gerüstatom Heteroatome sind, die ausgewählt sind aus der Gruppe Stickstoff, Schwefel oder Sauerstoff. Weiterhin können die carbocyclischen aromatischen Reste oder die heteroaromatischen Reste mit bis zu fünf gleichen oder verschiedenen Substituenten pro Cyclus substituiert sein, die ausgewählt sind aus der Gruppe Hydroxy, Chlor, Fluor, Nitro, Cyano, freies oder geschütztes Formyl, C₁-C₁₂-Alkyl, C₅-C₁₄-Aryl, C₆-C₁₅-Arylalkyl, -PO-[(C₁-C₈)-Alkyl]₂, -PO-[(C₅-C₁₄)-Aryl]₂, -PO-[(C₁-C₈)-Alkyl)(C₅-C₁₄)-Aryl)], Tri(C₁-C₈-alkyl)siloxyl oder Resten der Formel (VIIa-f),

A-B-D-E (VIIa) A-E (VIIb)

A-SO₂-E (VIIc) A-B-SO₂R⁴ (VIId)

A-SO₃W (VIIe) A-COW (VIIf)

in denen unabhängig voneinander
- A: fehlt oder für einen C₁-C₈-Alkylenrest steht und
- B: fehlt oder für Sauerstoff, Schwefel oder NR⁴ steht,
wobei R⁴ Wasserstoff, C₁-C₈-Alkyl, C₆-C₁₅-Arylalkyl oder C₅-C₁₄-Aryl bedeutet und
- D: für eine Carbonyl-Gruppe steht und
- E: für R⁵, OR⁵, NHR⁶ oder N(R⁶)₂ steht,
wobei R⁵ für C₁-C₈-Alkyl, C₆-C₁₅-Arylalkyl, C₁-C₈-Halogenalkyl oder C₅-C₁₄-Aryl und
R⁶ jeweils unabhängig für C₁-C₈-Alkyl, C₆-C₁₅-Arylalkyl oder C₅-C₁₄-Aryl oder N(R⁶)₂ zusammen für einen cyclischen Aminorest steht und
- W: für OH, NH₂, oder OM steht, wobei M ein Alkalimetallion, ein halbes Äquivalent eines Erdalkalimetallions, ein Ammoniumion oder ein organisches Ammoniumion bedeuten kann,
- Ar: steht besonders bevorzugt für Phenyl, Naphtyl, Phenanthrenyl, Anthracenyl, Biphenyl, Binaphthyl, Fluorenyl, Pyridinyl, Oxazolyl, Thiophen-yl, Benzofuranyl, Benzothiophenyl, Dibenzofuran-yl, Dibenzothiophen-yl, Furanyl, Indolyl, Pyridazinyl, Pyrazinyl, Pyrimidinyl, Triazolyl und Chinolinyl, wobei die genannten Reste weiterhin mit keinem, einem, zwei oder drei Resten pro Cyclus weiter substituiert sein können, die jeweils unabhängig voneinander ausgewählt sind aus der Gruppe Fluor, Nitro, Cyano, Di(C₁-C₄-alkyl)-amino, C₁-C₄-Alkyl, C₅-C₁₀-Aryl, C₁-C₈-Fluoralkyl, C₁-C₈-Fluoralkoxy, C₁-C₈-Alkoxy, CO(C₁-C₄-Alkyl), COO-(C₁-C₄)-Alkyl, -CON(C₁-C₄-Alkyl)₂,
- Ar: steht ganz besonders bevorzugt für einen Phenyl-Rest, der mit keinem, einem, zwei oder drei Resten weiter substituiert sein kann, die jeweils voneinander unabhängig ausgewählt sind aus der Gruppe Nitro, Fluor, Cyano, C₁-C₄-Alkyl, C₁-C₄-Alkoxy, Trifluormethyl, Trifluormethoxy, CO-(C₁-C₄)-Alkyl, COO-(C₁-C₄)-Alkyl und -CON(C₁-C₄-Alkyl)₂,
- n: steht bevorzugt für 1,
- Z: steht bevorzugt für Chlor, Brom oder Iod
- F: steht bevorzugt für Sauerstoff, Schwefel, NR³ oder Ethindiyl, wobei R³ für Wasserstoff, C₁-C₄-Alkyl oder C₅-C₁₈-Aryl steht,
- R²: steht bevorzugt für Ar oder C₁-C₁₂-Alkyl.

Für das erfindungsgemäße Verfahren werden die Verbindungen der Formel (I) im Allgemeinen in Mengen von 0,02 Mol% bis 10 Mol %, bevorzugt 0,1 Mol% bis 3 Mol%, bezogen auf die eingesetzten Verbindungen der Formel (IV), eingesetzt.

Als Base werden im erfindungsgemäßen Verfahren beispielsweise und bevorzugt Alkali- und/oder Erdalkalimetallcarbonate, -hydrogencarbonate, -alkoholate, -phosphate, -fluoride und/oder -hydroxide eingesetzt, wobei insbesondere Kalium- und/oder Natriumcarbonat, Cäsiumcarbonat, Cäsiumhydrogen-carbonat, Natriummethanolat, Kalium-tertiär-butylat, Kaliumamylat, Cäsiumfluorid, Kaliumphosphat und Bariumhydroxid zu nennen sind. Bevorzugt werden Kaliumcarbonat, Natriumcarbonat, Cäsiumcarbonat und/oder Cäsiumhydrogen-carbonat, eingesetzt.

Besonders bevorzugt wird Kaliumcarbonat eingesetzt.

Pro Mol auszutauschendem Hal in Verbindungen der Formel (IV) können beispielsweise 0,05 bis 10 Mol Base eingesetzt werden, bevorzugt 0,3 bis 2 Mol.

Für das erfindungsgemäße Verfahren ist es von Vorteil, wenn die eingesetzten Basen durch Mahlung und/oder Trocknung vorbehandelt werden.

Nach der Mahlung liegen die spezifischen Oberflächen der Basen vorzugsweise bei ca. 0,1 bis 10 m²/g, besonders bevorzugt bei 0,2 bis 1 m²/g (BET).

Aufgrund der ausgeprägten hygroskopischen Eigenschaften der im erfindungsgemäßen Verfahren eingesetzten Basen, neigen vor allem die Phosphate und Carbonate zur mehr oder minder starken Aufnahme atmosphärischer Bestandteile, wie Wasser und Kohlendioxid. Ab einer Aufnahme von ca. 30 Gewichtsprozent an atmosphärischen Bestandteilen ist ein deutlicher Einfluss auf die zu erreichenden Umsätze feststellbar. Daher ist neben der Mahlung häufig auch eine Trocknung der

Basen angezeigt.

Die Trocknung der Basen erfolgt dabei je nach Natur der verwendeten Base beispielsweise derart, dass unter vermindertem Druck von ca. 0,01 bis 100 mbar für mehrere Stunden auf Temperaturen von ca. 50 bis 200°C, bevorzugt 100 bis 160°C, erhitzt wird.

Das molare Verhältnis von Verbindungen der Formel (VI) zu Verbindungen der Formel (IV) kann beispielsweise 0,8 bis 10 betragen, bevorzugt 1 bis 6 und besonders bevorzugt 1 bis 4.

Das erfindungsgemäße Verfahren kann beispielsweise bei Temperaturen von 20 bis 250°C, bevorzugt bei 100 bis 150°C durchgeführt werden. Die optimalen Reaktionstemperaturen hängen dabei insbesondere von der Art der Ausgangsprodukte, des Katalysators und der eingesetzten Basen ab und lassen sich durch einfache Vorversuche ermitteln.

Das erfindungsgemäße Verfahren kann sowohl in Anwesenheit als auch in Abwesenheit eines geeigneten Lösungsmittels durchgeführt werden. Als Lösungsmittel kommen beispielsweise aliphatische, alicyclische oder aromatische Kohlenwasserstoffe, wie beispielsweise Benzin, Benzol, Toluol, Xylol, Petrolether, Hexan, Cyclohexan; Ether, wie Diethylether, Diisopropylether, Dioxan, Tetrahydrofuran oder Ethylenglykoldimethyl- oder -diethylether; Amide wie beispielsweise N,N-Dimethylformamid, N,N-Dimethylacetamid, N-Methylformanilid, N-Methylpyrrolidon oder Hexamethylphosphorsäuretriamid; Ester, wie Essigsäuremethylester oder Essigsäureethylester oder Mischungen solcher Lösungsmittel in Frage.

Gegebenenfalls kann auch ein Überschuss an Verbindungen der Formel (VI) als Reaktionsmedium dienen.

Dem erfindungsgemäßen Verfahren kann gegebenenfalls ein Schleppmittel zugesetzt werden, das gegebenenfalls bei der Reaktion entstehendes Wasser bei der Destillation kontinuierlich azeotrop entfernt.

Die Durchführung des erfindungsgemäßen Verfahrens kann nach üblichen Methoden in kontinuierlicher oder diskontinuierlicher Weise erfolgen.

Der Vorteil vorliegender Erfindung ist insbesondere die leichte Herstellung der Verbindungen der Formel (I) und die hohe Effizienz mit der die erfindungsgemäßen Kupferkomplexe zur Herstellung von Verbindungen der Formel (VI) eingesetzt werden können.

### Beispiele

### Herstellung von Verbindungen der Formel (I)

### Beispiel 1

### Herstellung von Bis(2-(di-tert-butylphosphino)biphenyl-kupfer(I)bromid)

50 ml entgastes, wasserfreies Methanol wurde auf Rückflusstemperatur erhitzt, 2,36 g (7,9 mmol) 2-(Di-tert.-butylphosphino)biphenyl wurde langsam dem Methanol zugegeben, bis die Phosphin-Verbindung vollständig gelöst war. Anschließend wurde 0,59 g (2,6 mmol) Kupfer(II)-bromid portionsweise der Lösung zugegeben. Nach Zugabe des Kupferbromids wurde die Lösung noch weitere 15 min lang auf Rückflusstemperatur erhitzt und danach die Lösung abgekühlt. Nach Abkühlen der Lösung fiel ein Feststoff aus, der abfiltriert wurde und mit wenig Ethanol und Diethylether gewaschen und anschließend getrocknet wurde. Man erhielt 0,93 g (1,1 mmol) der oben genannten Verbindung. Die Ausbeute betrug 80 % d. Th.

### Beispiel 2

### Herstellung von Bis(N-[2'-(dicyclohexylphosphino)-1,1'-biphenyl-2-yl]-N,N-dimethyl-aminkupfer(I)bromid)-Dimer

50 ml entgastes, wasserfreies Methanol wurde auf Rückflusstemperatur erhitzt. 2,00 g (5,1 mmol) N-[2'-(dicyclohexylphosphino)-1,1'-biphenyl-2-yl]-N,N-dimethylamine wurde langsam dem Methanol zugegeben, bis die Phosphin-Verbindung vollständig gelöst war. Anschließend wurde 0,8 g (3,7 mmol) Kupfer(II)-bromid portionsweise der Lösung zugegeben. Nach Zugabe des Kupferbromids wurde die Lösung noch weitere 15 min lang auf Rückflusstemperatur erhitzt und danach die Lösung abgekühlt. Nach Abkühlen der Lösung fiel ein Feststoff aus, der abfiltriert wurde und mit wenig Ethanol und Diethylether gewaschen und anschließend getrocknet wurde. Man erhielt 1,5 g der oben genannten Verbindung (1,4mmol, M = 1073,8 g/mol). Die Struktur wurde über eine FD-MS Messung (m/e=1074) überprüft. Die Ausbeute betrug 73 % d. Th.

### Beispiel 3

### Herstellung von 3-[(Diphenylphosphino)oxy]phenyl diphenylphosphonit-kupfer(I)chlorid

In einem Rundkolben werden 5 g (10,4 mmol) 3-[(Diphenylphosphino)oxy]phenyl diphenylphosphonit in entgastem, wasserfreiem Dichlormethan gelöst und auf 40°C erwärmt. 0,35 g Kupfer(I)chlorid (0,35 mmol) werden zugegeben. Nach 30 Minuten rühren wird das Lösungsmittel im Vakuum abgezogen. Erhalten wurde die o.g. Verbindung.

### Beispiel 4

### Herstellung von Bis-(1,1'-biphenyl-2-yl-dicyclohexylphosphonit-kupfer(I)chlorid]

In einem Rundkolben werden 5 g (13,6 mmol) 1,1'-Biphenyl-2-yl-dicyclohexylphosphonit in wasserfreiem, entgastem Chloroform gelöst. Im Argongegenstrom werden 0,45 g (4,5mmol) Kupfer(I)chlorid zugegeben und 6 h bei Raumtemperatur gerührt. Das Lösungsmittel wird im Vakuum entfernt und der Rückstand in wasserfreiem Ether aufgenommen. Man kühlt auf -78°C ab, dabei fällt das Produkt aus.

### Beispiel 5

### Herstellung von Bis-[4-isopropoxydinapbtho[2,1-d:1',2'-f][1,3,2]dioxa-phosphepial-Kupfer(1) chlorid

In einem Rundkolben werden 5 g (13,4 mmol) 4-Isopropoxydinaphtho(2,1-d:1',2'-f][1,3,2]dioxaphosphepin (rac.-Binaphthyl-isopropyl-phosphit) in Dichlormethan gelöst und im Argongegenstrom 0,44 g (0,45 mmol) Kupfer(I)chlorid zugegeben. Nach 30 Minuten rühren wird das Lösungsmittel im Vakuum abgezogen.

### Beispiele 6 bis 24

### Verwendung der Katalysatoren der Beispiele 1 und 2 in Kupplungsreaktionen

### Beispiel 6

### Kupplung von p-Bromacetophenon mit n-Octanthiol (Katalysator aus Beispiel 1)

1,35 g (6,7 mmol) p-Bromacetophenon, 1,0 g (6,7 mmol) n-Octanthiol, 1,8 g (13,5 mmol) Kaliumcarbonat und 300 mg (0,7 mmol) des Katalysators aus Beispiel 1 werden in 50 ml Dioxan unter Argonatmosphäre 12 h bei 110°C gerührt. Die Reaktionslösung wird anschließend mit 40 ml wässrigem Ammoniak versetzt, mit Essigsäureethylester extrahiert und die vereinigten organischen Extrakte im Vakuum getrocknet. Nach säulenchromatographischer Aufarbeitung (Hexan) werden 1,24 g (70 %) Produkt erhalten.
GC-MS/EI: 264 (M)

### Beispiel 7

### Kupplung von p-Bromacetophenon mit n-Octanthiol (Katalysator aus Beispiel 2)

1,35 g (6,7 mmol) p-Bromacetophenon, 1,0 g (6,7 mmol) n-Octanthiol, 1,8 g (13,5 mmol) Kaliumcarbonat und 360 mg (0,7 mmol) des Katalysators aus Beispiel 2 werden in 50 ml Dioxan unter Argonatmosphäre 12 h bei 110°C gerührt. Die Reaktionslösung wird anschließend mit 40 ml wässrigem Ammoniak versetzt, mit Essigsäureethylester extrahiert und die vereinigten organischen Extrakte im Vakuum getrocknet. Nach säulenchromatographischer Aufarbeitung (Hexan) werden 733 mg (40 %) Produkt erhalten.

### Beispiel 8

### Kupplung von p-Bromacetophenon mit Thiophenol (Katalysator aus Beispiel 1)

1,35 g (6,7 mmol) p-Bromacetophenon, 0,75 g (6,7 mmol) Thiophenol, 1,8 g (13,5 mmol) Kaliumcarbonat und 300 mg (0.7 mmol) des Katalysators aus Beispiel 1 werden in 50 ml Dioxan unter Argonatmosphäre 12 h bei 110°C gerührt. Die Reaktionslösung wird anschließend mit 20 ml wässrigem Ammoniak versetzt, mit Essigsäureethylester extrahiert und die vereinigten organischen Extrakte im Vakuum getrocknet. Nach säulenchromatographischer Aufarbeitung (Hexan) werden 970 mg (65%) Produkt erhalten.
GC-MS/EI: 228 (M)

### Beispiel 9

### Kupplung von p-Bromacetophenon mit Thiophenol (Katalysator aus Beispiel 2)

1,35 g (6,7 mmol) p-Bromacetophenon, 0,75 g (6,7 mmol) Thiophenol, 1,8 g (13,5 mmol) Kaliumcarbonat und 360 mg (0,7 mmol) des Katalysators aus Beispiel 2 werden in 50 ml Dioxan unter Argonatmosphäre 12 h bei 110°C gerührt. Die GC-Analyse des Rohproduktes zeigt die Produktbildung mit 75 % Umsatz an.

### Beispiel 10

### Kupplung von p-Bromacetophenon mit Phenol (Katalysator aus Beispiel 1)

1,35 g (6,7 mmol) p-Bromacetophenon, 630 mg (6,7 mmol) Phenol, 1,8 g (13,5 mmol) Kaliumcarbonat und 300 mg (0,7 mmol) des Katalysators aus Beispiel 1 werden in 50 ml Dioxan unter Argonatmosphäre 12 h bei 110°C gerührt. Die Reaktionslösung wird anschließend mit 40 ml wässrigem Ammoniak versetzt, mit Essigsäureethylester extrahiert und die vereinigten organischen Extrakte im Vakuum getrocknet. Nach säulenchromatographischer Aufarbeitung (Hexan) werden 280 mg (20 %) Produkt erhalten.
GC-MS/EI: 212

### Beispiel 11

### Kupplung von p-Bromacetophenon mit Phenol (Katalysator aus Beispiel 2)

1,35 g (6,7 mmol) p-Bromacetophenon, 630 mg (6,7 mmol) Phenol, 1,8 g (13,5 mmol) Kaliumcarbonat und 360 mg (0,7 mmol) des Katalysators aus Beispiel 2 werden in 50 ml Dioxan unter Argonatmosphäre 12 h bei 110°C gerührt. Die GC-Analyse des Rohproduktes zeigt die Produktbildung mit 18 % Umsatz an.

### Beispiel 12

### Kupplung von p-Bromacetophenon mit 2-Hydroxypyridin

### (Katalysator aus Beispiel 1)

1,35 g (6,7 mmol) p-Bromacetophenon, 650 mg (6,7 mmol) 2-Hydroxypyridin, 1,8 g (13,5 mmol) Kaliumcarbonat und 300 mg (0,7 mmol) des Katalysators aus Beispiel 1 werden in 50 ml Dioxan unter Argonatmosphäre 12 h bei 110°C gerührt. Die Reaktionslösung wird anschließend mit 40 ml wässrigem Ammoniak versetzt, mit Essigsäureethylester extrahiert und die vereinigten organischen Extrakte im Vakuum getrocknet. Nach säulenchromatographischer Aufarbeitung (Hexan) werden 740 mg (52 %) eines Produktgemisches von N-4-acetylphenyl-pyridinon und 4-Acetylphenoxy-2-pyridin (Verhältnis 6:1) erhalten.
GC-MS/EI: 213 (M)

### Beispiel 13

### Kupplung von p-Bromacetophenon mit 2-Hydroxypyridin

### (Katalysator aus Beispiel 2)

1,35 g (6,7 mmol) p-Bromacetophenon, 650 mg (6,7 mmol) o-Hydroxypyridin, 1,8 g (13,5 mmol) Kaliumcarbonat und 360 mg (0,7 mmol) des Katalysators aus Beispiel 2 werden in 50 ml Dioxan unter Argonatmosphäre 12 h bei 110°C gerührt. Die GC-Analyse des Rohproduktes zeigt die Produktbildung mit 42 % Umsatz an.

### Beispiel 14

### Kupplung von p-Bromacetophenon mit Methanol (Katalysator aus Beispiel 1)

1,34 g (13,5 mmol) p-Bromacetophenon, 2 ml (20 mmol) Essigsäureethylester, 17,5 ml einer 30 %igen Natriununethylatlösung und 300 mg (0,7 mmol) des Katalysators aus Beispiel 1 werden unter Argonatmosphäre 12 h refluxiert. Die Reaktionslösung wird anschließend vorsichtig hydrolysiert, mit Dichlormethan extrahiert und die vereinigten organischen Extrakte im Vakuum getrocknet. Im GC sind neben dem Produkt (35%) mit hohem Anteil Fragmente des Aldol-Nebenproduktes zu sehen. Nach säulenchromatographischer Aufarbeitung (Hexan) wurden 820 mg (41 %) Produkt erhalten.
GC-MS/EI: 150 (M)

### Beispiel 15

### Kupplung von p-Bromacetophenon mit Methanol (Katalysator aus Beispiel 2)

1,34 g (13,5 mmol) p-Bromacetophenon, 2 ml (20 mmol) Essigsäureethylester, 17,5 ml einer 30 %igen Natriummethylatlösung und 360 mg (0,7 mmol) des Katalysators aus Beispiel 2 werden unter Argonatmosphäre 12 h refluxiert. Die Reaktionslösung wird anschließend vorsichtig hydrolysiert, mit Dichlormethan extrahiert und die vereinigten organischen Extrakte im Vakuum getrocknet. Im GC sind neben dem Produkt (28 %) mit hohem Anteil Fragmente des Aldol-Nebenproduktes zu sehen.

### Beispiel 16

### Kupplung von 4-Jodtrifluormethylbenzol mit Phenylacetylen

### (Katalysator aus Beispiel 1)

2,7 g (10 mmol) 4-Jodtrifluormethylbenzol, 1,3 g (12,5 mmol) Phenylacetylen, 2,2 g (20 mmol) Kalium-tert-butylat und 300 mg (0,7 mmol) des Katalysators aus Beispiel 1 werden in 100 ml Dioxan unter Argonatmosphäre 21 h bei 110°C gerührt. Die Reaktionslösung wird filtriert und im Vakuum getrocknet. Nach säulenchromatographischer Aufarbeitung (Hexan) werden 1,88 g (75 %) Produkt erhalten.
GC-MS/EI: 246 (M)

### Beispiel 17

### Kupplung von 4-Jodtrifluormethylbenzol mit Phenylacetylen

### (Katalysator aus Beispiel 2)

2,7 g (10 mmol) 4-Jodtrifluormethylbenzol, 1,3 g (12,5 mmol) Phenylacetylen, 2,2 g (20 mmol) Kalium-tert-butylat und 360 mg (0,7 mmol) des Katalysators aus Beispiel 2 werden in 100 ml Dioxan unter Argonatmosphäre 21 h bei 110°C gerührt. Die GC-Analyse des Rohproduktes zeigt die Produktbildung (vgl. mit GC-MS/EI GZN 283-4) mit 62 % Umsatz an.

### Beispiel 18

### Kupplung von p-Tolyljodid mit Phenylacetylen (Katalysator aus Beispiel 1)

2,2 g (10 mmol) p-Tolyljodid, 1,3 g (12,5 mmol) Phenylacetylen, 2,2 g (20 mmol) Kalium-tert-butylat und 300 mg (0,7 mmol) des Katalysators aus Beispiel 1 werden in 100 ml Dioxan unter Argonatmosphäre 21 h bei 110°C gerührt. Die Reaktionslösung wird filtriert und im Vakuum getrocknet. Nach säulenchromatographischer Aufarbeitung (Hexan) werden 1.75 g (91 %) Produkt erhalten.
GC-MS/EI: 192 (M)

### Beispiel 19

### Kupplung von p-Tolyljodid mit Phenylacetylen atalysator aus Beispiel 2)

2,2 g (10 mmol) p-Tolyljodid, 1,3 g (12.5 mmol) Phenylacetylen, 2,2 g (20 mmol) Kalium-tert-butylat und 360 mg (0,7 mmol) des Katalysators aus Beispiel 2 werden in 100 ml Dioxan unter Argonatmosphäre 21 h bei 110°C gerührt. Die GC-Analyse des Rohproduktes zeigt die Produktbildung (vgl. mit GC-MS/EI GZN 277-8) mit 50 % Umsatz an.

### Beispiel 20

### Kupplung von Brombenzol mit Anilin (Katalysator aus Beispiel 1)

1,05 g (6,7 mmol) Brombenzol, 1,4 g (10,1 mmol) Cäsiumcarbonat und 300 mg (0,7 mmol) des Katalysators aus Beispiel 1 werden in 2 ml Anilin unter Argonatmosphäre 12 h bei 170°C gerührt. Die GC-Analyse des Rohproduktes zeigt die selektive Bildung der monoarylierten Verbindung (GC-MS/EI: 169 (M)) und einem Umsatz von 5% an, Triarylamin wird nicht detektiert.

### Beispiel 21

### Kupplung von Jodbenzol mit Anilin (Katalysator aus Beispiel 1)

1,37 g (6,7 mmol) Jodbenzol, 1,4 g (10,1 mmol) Cäsiumcarbonat und 300 mg (0,7 mmol) des Katalysatore aus Beispiel 1 werden in 2 ml Anilin unter Argonatmosphäre 12 h bei 170°C gerührt. Die GC-Analyse des Rohproduktes zeigt die selektive Bildung der monoarylierten Verbindung (Vergleich mit GC-MS/EI von 1126-7) an, Triarylamin wird nicht detektiert. Nach säulenchromatographischer Aufarbeitung werden 810 mg (72 %) Produkt erhalten.

### Beispiel 22

### Kupplung von p-Chlornitrobenzol mit Anilin (Katalysator aus Beispiel 1)

1,06 g (6,7 mmol) p-Chlornitrobenzol, 1,4 g (10,1 mmol) Cäsiumcarbonat und 300 mg (0,7 mmol) des Katalysators aus Beispiel 1 werden in 2 ml Anilin unter Argonatmosphäre 12 h bei 170°C gerührt. Die GC-Analyse des Rohproduktes zeigt die selektive Bildung der monoarylierten Verbindung und 83 % Umsetzung an, Triarylamin wird nicht detektiert. Nach säulenchromatographischer Aufarbeitung werden 1,14 g (80 %) Produkt erhalten.
GC-MS/EI: 214 (M)

### Beispiel 23

### Kupplung von Jodbenzol mit Anilin (Katalysator aus Beispiel 2)

1,37 g (6,7 mmol) Jodbenzol, 1,4 g (10,1 mmol) Cäsiumcarbonat und 360 mg (0,7 mmol) des Katalysators aus Beispiel 2 werden in 2 ml Anilin unter Argonatmosphäre 12 h bei 170°C gerührt. Die GC-Analyse des Rohproduktes zeigt die selektive Bildung der monoarylierten Verbindung und vollständige Umsetzung an, Triarylamin wird nicht detektiert.

### Beispiel 24

### Kupplung von p-Chlornitrobenzol mit Anilin (Katalysator aus Beispiel 2)

1,06 g (6,7 mmol) p-Chlornitrobenzol, 1,4 g (10,1 mmol) Cäsiumcarbonat und 360 mg (0,7 mmol) des Katalysators aus Beispiel 2 werden in 2 ml Anilin unter Argonatmosphäre 12 h bei 170°C gerührt. Die GC-Analyse des Rohproduktes zeigt die selektive Bildung der monoarylierten Verbindung (Vergleich mit GC-MS/EI von 1101-7) und 91% Umsetzung an, Triarylamin wird nicht detektiert.

## Patentansprüche

1. Verfahren zur Herstellung von Verbindungen der Formel (IV),
Ar-(F-R²)ₙ (IV)
in der
n für 1, 2 oder 3 steht und
Ar für einen substituierten oder unsubstituierten aromatischen Rest und
F für Sauerstoff, Schwefel, NR³, NR³CO oder Ethindiyl steht, wobei R³ für Wasserstoff, C₁-C₁₂-Alkyl, C₅-C₁₈-Aryl oder C₆-C₁₉-Arylalkyl steht und
R² für Ar, C₁-C₁₂-Alkyl, C₁-C₁₂-Halogenalkyl, C₂-C₁₂-Alkenyl oder C₆-C₁₉-Arylalkyl steht,
**dadurch gekennzeichnet, dass** Verbindungen der Formel (V)
Ar-Z (V)
in der
Ar vorstehend genannte Bedeutung besitzt und
Z für Chlor, Brom, Iod, ein Diazoniumsalz oder ein Sulfonat steht
mit Verbindungen der Formel (VI) umgesetzt werden,
H-F-R² (VI)
in der
F und R² die vorstehend genannte Bedeutung besitzen und
wobei die Umsetzung in Gegenwart von Base und Verbindungen der Formel (I) erfolgt, 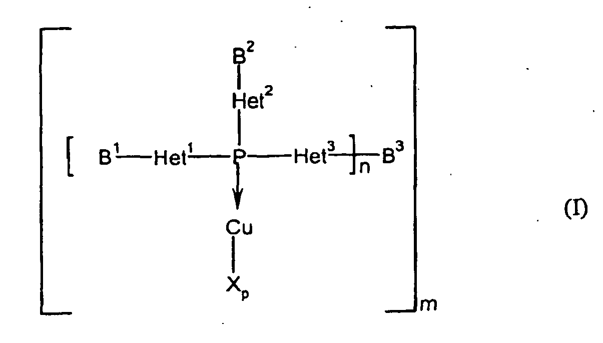 worin
Het¹, Het² und Het³ jeweils unabhängig voneinander fehlen, für Sauerstoff oder NR¹ stehen, wobei R¹ für C₁-C₈-Alkyl, C₅-C₁₈-Aryl oder C₆-C₁₉-Arylalkyl steht und
B¹ und B² jeweils unabhängig voneinander für C₁-C₈-Alkyl, C₅-C₁₈-Aryl oder C₆-C₁₉-Arylalkyl stehen oder wobei die Reste B¹ und B² gemeinsam für einen divalenten Rest mit insgesamt 2 bis 40 Kohlenstoffatomen stehen können und
B³ für C₁-C₈-Alkyl, C₅-C₁₈-Aryl, C₆-C₁₉-Arylalkyl oder einen Rest mit insgesamt 2 bis 40 Kohlenstoffatomen und der Wertigkeit n steht,
X für Halogenid, (C₁-C₈-Halogenalkyl)carboxylat, (C₁-C₈-Alkyl)carboxylat, (C₁-C₈-Halogenalkyl)sulfonat, (C₅-C₁₈-Aryl)sulfonat, Cyanid, gegebenenfalls fluoriertes Acetylacetonat, Nitrat, Oxinat, Phosphat, Carbonat, Hexafluorophosphat, Tetraphenylborat, Tetrakis(pentafluorphenyl)borat oder Tetrafluoroborat steht, und
p für 0, 1 oder 2 steht und
n für 1, 2 oder 3 steht und
m für 1, 2 , 3, 4, 5 oder 6 steht.

2. Verfahren nach Anspruch 1, **dadurch gekennzeichnet, dass** die Verbindungen der Formel (I) als isolierte Verbindungen eingesetzt oder in situ erzeugt werden.

3. Verfahren nach mindestens einem der Ansprüche 1 und 2, **dadurch gekennzeichnet, dass** Ar für carbocyclische aromatische Reste mit 6 bis 24 Gerüstkohlenstoffatomen oder für heteroaromatische Reste mit 5 bis 24 Gerüstatomen, in denen keines, ein, zwei oder drei Gerüstatome pro Zyklus, im gesamten Molekül mindestens jedoch ein Gerüstatom Heteroatome sind, die ausgewählt sind aus der Gruppe Stickstoff, Schwefel oder Sauerstoff und
wobei die carbocyclischen aromatischen Reste oder die heteroaromatischen Reste mit bis zu fünf gleichen oder verschiedenen Substituenten pro Zyklus substituiert sein können, die ausgewählt sind aus der Gruppe Hydroxy, Chlor, Fluor, Nitro, Cyano, freies oder geschütztes Formyl, C₁-C₁₂-Alkyl, C₅-C₁₄-Aryl, C₆-C₁₅-Arylalkyl, -PO-[(C₁-C₈)-Alkyl]₂, -PO-[(C₅-C₁₄)-Aryl]₂, -PO-[(C₁-C₈)-Alkyl)(C₅-C₁₄)-Aryl)], Tri(C₁-C₈-alkyl)siloxyl oder Resten der Formel (VIIa-f),
A-B-D-E (VIIa) A-E (VIIb)
A-SO₂-E (VIIc) A-B-SO₂R⁴ (VIId)
A-SO₃W (VIIe) A-COW (VIIf)
in denen unabhängig voneinander
A fehlt oder für einen C₁-C₈-Alkylenrest steht und
B fehlt oder für Sauerstoff, Schwefel oder NR⁴ steht,
wobei R⁴ Wasserstoff, C₁-C₈-Alkyl, C₆-C₁₅-Arylalkyl oder C₅-C₁₄-Aryl bedeutet und
D für eine Carbonyl-Gruppe steht und
E für R⁵, OR⁵, NHR⁶ oder N(R⁶)₂ steht,
wobei R⁵ für C₁-C₈-Alkyl, C₆-C₁₅-Arylalkyl, C₁-C₈-Halogenalkyl oder C₅-C₁₄-Aryl und
R⁶ jeweils unabhängig für C₁-C₈-Alkyl, C₆-C₁₅-Arylalkyl oder C₅-C₁₄-Aryl oder N(R⁶)₂ zusammen für einen cyclischen Aminorest steht und
W für OH, NH₂, oder OM steht, wobei M ein Alkalimetallion, ein halbes Äquivalent eines Erdalkalimetallions, ein Ammoniumion oder ein organisches Ammoniumion bedeuten kann.

4. Verfahren nach mindestens einem der Ansprüche 1 bis 3, **dadurch gekennzeichnet, dass** R² für Ar oder C₁-C₁₂-Alkyl steht.

5. Verfahren nach mindestens einem der Ansprüche 1 bis 4, **dadurch gekennzeichnet, dass** die Verbindungen der Formel (I) in Mengen von 0,02 Mol% bis 10 Mol % bezogen auf die eingesetzten Verbindungen der Formel (IV) eingesetzt werden.

6. Verfahren nach mindestens einem der Ansprüche 1 bis 5, **dadurch gekennzeichnet, dass** als Base Alkali- und/oder Erdalkalimetallcarbonate, -hydrogencarbonate, -alkoholate, -phosphate, -fluoride und/oder -hydroxide eingesetzt werden.

7. Verfahren nach mindestens einem der Ansprüche 1 bis 6, **dadurch gekennzeichnet, dass** die eingesetzten Basen durch Mahlung und/oder Trocknung vorbehandelt werden.

8. Verfahren nach mindestens einem der Ansprüche 1 bis 7, **dadurch gekennzeichnet, dass** die Umsetzung in Gegenwart von Verbindungen der Formel (I) erfolgt, worin Het¹, Het² und Het³ jeweils unabhängig voneinander für Sauerstoff stehen oder fehlen.

9. Verfahren nach mindestens einem der Ansprüche 1 bis 8, **dadurch gekennzeichnet, dass** die Umsetzung in Gegenwart von Verbindungen der Formel (I) erfolgt, worin B¹ und B² jeweils unabhängig voneinander für sekundäres oder tertiäres C₃-C₈- bzw. C₄-C₈-Alkyl, C₅-C₁₈₋Aryl oder Bis-(C₅-C₁₈-Aryl stehen oder B¹ und B² gemeinsam für einen divalenten Rest stehen, der ausgewählt ist aus der Gruppe 1,2-Phenylen, 1,3-Phenylen, 1,2-Cyclohexyl, 1,1'-Ferrocenyl, 1,2-Ferrocenyl, 2,2'-(1,1'-Binaphtyl) und 1,1'-Biphenyl, wobei die genannten Reste gegebenenfalls einfach oder mehrfach durch Cyano, Chlor, Fluor, C₁-C₁₂-Alkyl, C₁-C₁₂₋Halogenalkyl, C₁-C₁₂-Alkoxy, C₁-C₁₂-Halogenalkoxy, Di(C₁-C₈-alkyl)amino oder Tri(C₁-C₈₋alkyl)siloxyl substituiert sein können.

10. Verfahren nach mindestens einem der Ansprüche 1 bis 9, **dadurch gekennzeichnet, dass** die Umsetzung in Gegenwart von Verbindungen der Formel (I) erfolgt, worin B³ für sekundäres oder tertiäres C₃-C₈- bzw. C₄-C₈-Alkyl, C₅-C₁₈-Aryl, C₆-C₁₉-Arylalkyl oder einen Rest mit insgesamt 2 bis 40 Kohlenstoffatomen und der Wertigkeit n steht.

11. Verfahren nach mindestens einem der Ansprüche 1 bis 10, **dadurch gekennzeichnet, dass** die Umsetzung in Gegenwart von Verbindungen der Formel (I) erfolgt, worin X für Chlorid; Bromid oder Iodid, Trifluormethansulfonat, Trifluoracetat, Methansulfonat, Benzolsulfonat, Cyanid, gegebenenfalls fluoriertes Acetylacetonat, Hexafluorophosphat oder Tetrafluoroborat steht.

12. Verfahren nach mindestens einem der Ansprüche 1 bis 11, **dadurch gekennzeichnet, dass** die Umsetzung in Gegenwart von Verbindungen der Formel (I) erfolgt, worin n für 1 oder 2 und m für 1 oder 2 steht.

13. Verfahren nach mindestens einem der Ansprüche 1 bis 12, **dadurch gekennzeichnet, dass** die Umsetzung in Gegenwart von Verbindungen der Formel (I) erfolgt, die folgende Phosphorverbindungen als Liganden enthalten: Bis(2-dicyclohexylphosphino)-2'-(N,N-dimethyl-amino)-biphenyl, 2-(Dicyclohexylphosphino)biphenyl, 2-(Dicyclohexylphosphino)-2'-methylbiphenyl, 2-(Di-tert.-butylphosphino)biphenyl, 2-(Bisdiphenylphosphino)binaphthyl, 1,1'-Biphenyl-2-yl-dicyclohexylphosphonit, 1, 1'-Biphenyl-2-yl-ditert-butyiphosphonit, 3-[(diisopropylphosphino)oxy]phenyldiisopropylphosphonit, 3-[(Di-tert.-butylphosphino)oxy]phenyl-di-tert.-butyl-phosphonit, 3-[(Diphenylphosphino)oxy]phenyl-diphenylphosphonit, 3-[(dicyclohexylphosphino)oxy]phenyl-dicyclohexylphosphonit, 1,1'-Binaphthyl-2,2'-diyl-iso-propyl-phosphit, Tris(2,4-di-tertiär-butylphenyl)phosphit und 2,4,8,10-Tetra-tert.-butyl-6-phenoxy-12H-dibenzo[d,g] [1,3,2]dioxaphosphocin.

14. Verfahren nach mindestens einem der Ansprüche 1 bis 13, **dadurch gekennzeichnet, dass** die Umsetzung in Gegenwart von Verbindungen der Formel (I) ausgewählt aus [(µ-Br)₂{2-(di-tertiär-butylphosphino)biphenyl) ₂Cu₂], [(µ-Trifluormethansulfonato)₂ {2-(di-tertiärbutylphosphino)biphenyl}₂Cu₂], [(u-Br)₂{2-(dicyclohexylphosphino)biphenyl}₂Cu₂], [(µ-Trifluormethansulfonato)₂{2-(dicyclohexylphosphino)biphenyl}₂Cu₂] und [(µ-Br)₂{1,1'-Binaphthyl-2,2'-diyl-iso-propyl-phosphit)2Cu₂) erfolgt.

15. Verbindungen der Formel (I), 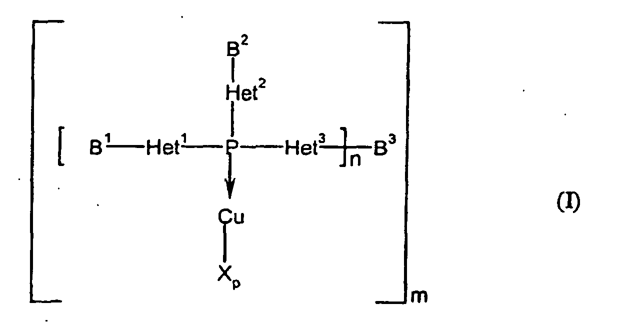 worin
Het¹, Het² und Het³ jeweils unabhängig voneinander fehlen, für Sauerstoff oder NR¹ stehen, wobei R¹ für C₁-C₈-Alkyl, C₅-C₁₈-Aryl oder C₆-C₁₉-Arylalkyl steht und
B¹ und B² jeweils unabhängig voneinander für C₁-C₈-Alkyl, C₅-C₁₈-Aryl oder C₆-C₁₉-Arylalkyl stehen oder wobei die Reste B¹ und B² gemeinsam für einen divalenten Rest mit insgesamt 2 bis 40 Kohlenstoffatomen stehen können und
X für Halogenid, (C₁-C₈-Halogenalkyl)carboxylat, (C₁-C₈-Alkyl)carboxylat, (C₁-C₈-Halogenaikyl)sulfonat, (C₅-C₁₈-Aryl)sulfonat, Cyanid, gegebenenfalls fluoriertes Acetylacetonat, Nitrat, Oxinat, Phosphat, Carbonat, Hexafluorophosphat, Tetraphenylborat, Tetrakis(pentafluorphenyl)borat oder Tetrafluoroborat steht, und
p für 0, 1 oder 2 steht und
n fiir 1, 2 oder 3 steht und
m für 1, 2, 3, 4, 5 oder 6 steht.
**dadurch gekennzeichnet, dass** sie folgende Phosphorverbindungen als Liganden enthalten: Bis(2-dicyclohexylphosphino)-2'-(N,N-dimethyl-amino)-biphenyl, 2-(Dicyclohexylphosphino)-biphenyl, 2-(Dicyclohexylphosphino)-2'-methylbiphenyl, 2-(Di-tert.-butylphosphino)-biphenyl, 2-(Bisdiphenylphosphino)binaphthyl, 1,1'-Biphenyl-2-yl-dicyclohexylphosphonit, 1,1'-Biphenyl-2-yl-ditert.-butylphosphonit, 3-[(diisopropylphosphino)oxy]phenyldiisopropylphosphonit, 3-[(Di-tert.-butyl-phosphino)oxy]phenyl-di-tert.-butyl-phosphonit, 3-[(Diphenylphosphino)oxy]phenyl-diphenylphosphonit, 3-[(dicyclohexylphosphino)oxy]phenyl-dicyclohexylphosphonit, 1,1'-Binaphthyl-2,2'-diyl-iso-propylphosphit, Tris(2,4-di-tertiär-butylphenyl)phosphit und 2,4,8,10-Tetra-tert.-butyl-6-phenoxy-12H-dibenzo[d,g] [1,3,2]dioxaphosphocin.

16. [(µ-Br)₂{2-(di-tertiär-butylphosphino)biphenyl}₂Cu₂], [(µ-Thrifluormethansulfonato)₂{2-(di-tertiär-butylphosphino)biphenyl}₂Cu₂], [(µ-Br)₂{2-(dicyclohexylphosphino)biphenyl}₂Cu₂], [(µ-Trifluormethansulfonato)₂{2-(dicyclohexylphosphino)biphenyl}₂Cu₂] und [(µ-Br)₂{1,1'-Binaphthyl-2,2'-diyl-iso-propyl-phosphit)₂Cu₂].

17. Verwendung von Verbindungen nach mindestens einem der Ansprüche 15 und 16 zur Knüpfung von Kohlenstoff-Stickstoff-, Kohlenstoff-Sauerstoff und Kohlenstoff-Schwefelbindungen sowie zur Herstellung von Arylalkinen.

18. Katalysatoren enthaltend Verbindungen nach mindestens einem der Ansprüche 15 und 16.

## Claims

1. Process for preparing compounds of the formula (IV)
Ar-(F-R²)ₙ (IV)
in which
n is 1, 2 or 3 and
Ar is a substituted or unsubstituted aromatic radical and
F is oxygen, sulphur, NR³, NR³CO or ethyndiyl, where R³ is hydrogen, C₁-C₁₂-alkyl, C₅-C₁₈-aryl or C₆-C₁₉-arylalkyl and
R² is Ar, C₁-C₁₂-alkyl, C₁-C₁₂-baloalkyl, C₂-C₁₂-alkenyl or C₆-C₁₉-arylalkyl,
**characterized in that** compounds of the formula (V)
Ar-Z (V)
in which
Ar is as defined above and
Z is chlorine, bromine, iodine, a diazonium salt or sulphonate
are reacted with compounds of the formula (VI)
H-F-R² (VI)
in which
F and R² are each as defined above and
wherein the conversion is effected in the presence of base and compounds of the formula (1) 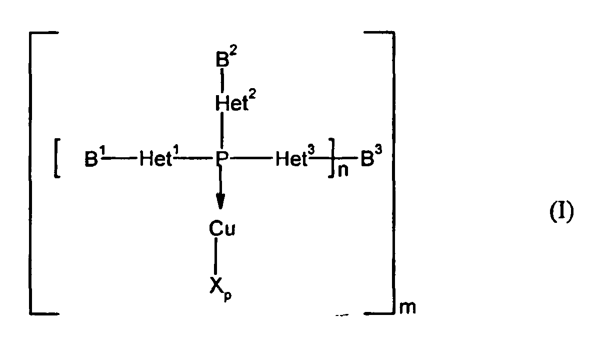 in which
Het¹, Het² and Bet³ are each independently absent, or are oxygen or NR¹ where R¹ is C₁-C₈-alkyl, C₅-C₁₈-aryl or C₆-C₁₉-arylalkyl and
B¹ and B² are each independently C₁-C₈-alkyl, C₅-C₁₈-aryl or C₆-C₁₉-arylalkyl, or the B¹ and B² radicals together may be a divalent radical having a total of 2 to 40 carbon atoms and
B³ is C₁-C₈-alkyl, C₅-C₁₈-aryl, C₆-C₁₉-arylalkyl or a radical having a total of 2 to 40 carbon atoms and the valency n,
X is halide, (C₁-C₈-haloalkyl)carboxylate, (C₁-C₈-alkyl)carboxylate, (C₁-C₈-haloalkyl)sulphonate, (C₅-C₁₈-aryl)sulphonate, cyanide, optionally fluorinated acetylacetonate, nitrate, oxinate, phosphate, carbonate, hexafluorophosphate, tetraphenylborate, tetrakis-(pentafluorophenyl)borate or tetrafluoroborate, and
p is 0, 1 or 2 and
n is 1, 2 or 3 and
m is 1, 2 , 3, 4, 5 or 6.

2. Process according to Claim 1, **characterized in that** the compounds of the formula (I) are used as isolated compounds or generated in situ.

3. Process according to at least one of Claims 1 and 2, **characterized in that** Ar is carbocyclic aromatic radicals having 6 to 24 framework carbon atoms or heteroaromatic radicals having 5 to 24 framework atoms of which no, one, two or three framework atoms per cycle, but at least one framework atom in the entire molecule, are heteroatoms which are selected from the group of nitrogen, sulphur and oxygen, and the carbocyclic aromatic radicals or the heteroaromatic radicals may be substituted by up to five identical or different substituents per cycle which are selected from the group of hydroxyl, chlorine, fluorine, nitro, cyano, free or protected formyl, C₁-C₁₂₋alkyl, C₅-C₁₄-aryl, C₆-C₁₅-arylalkyl, -PO-[(C₁-C₈)-alkyl]₂, -PO-[(C₅-C₁₄)-aryl]₂, -PO-[(C₁-C₈)-alkyl)(C₅-C₁₄)aryl)], tri(C₁-C₈-alkyl)siloxyl or radicals of the formula (VIIa-f)
A-B-D-E (VIIa) A-E (VIIb)
A-SO₂-E (VIIc) A-B-SO₂R⁴ (VIId)
A-SO₃W (VIIe) A-COW (VIIf)
in which, each independently,
A is absent or is a C₁-C₈-alkylene radical and
B is absent or is oxygen, sulphur or NR⁴,
where R⁴ is hydrogen, C₁-C₈-alkyl, C₆-C₁₅-arylalkyl or C₅-C₁₄-aryl and
D is a carbonyl group and
E is R⁵, OR⁵, NHR⁶ or N(R⁶)₂,
where R⁵ is C₁-C₈-alkyl, C₆-C₁₅-arylalkyl, C₁-C₈-haloalkyl or C₅-C₁₄-aryl and
R⁶ is in each case independently C₁-C₈-alkyl, C₆-C₁₅-arylalkyl or C₅-C₁₄-aryl, or N(R⁶)₂ together is a cyclic amino radical and
W is OH, NH₂ or OM where M may be an alkali metal ion, half an equivalent of an alkaline earth metal ion, an ammonium ion or an organic ammonium ion.

4. Process according to at least one of Claims 1 to 3, **characterized in that** R² is Ar or C₁-C₁₂-alkyl.

5. Process according to at least one of Claims 1 to 4, **characterized in that** the compounds of the formula (I) are used in amounts of 0.02 mol% to 10 mol%, based on the compounds of the formula (IV) used.

6. Process according to at least one of Claims 1 to 5, **characterized in that** the base used is an alkali metal and/or alkaline earth metal carbonate, hydrogencarbonate, alkoxide, phosphate, fluoride and/or hydroxide.

7. Process according to at least one of Claims 1 to 6, **characterized in that** the bases used are pretreated by grinding and/or drying.

8. Process according to at least one of Claims 1 to 7, **characterized in that** the reaction is effected in the presence of compounds of the formula (I) in which Het¹, Het² and Het³ are each independently oxygen or are absent.

9. Process according to at least one of Claims 1 to 8, **characterized in that** the reaction is effected in the presence of compounds of the formula (I) in which B¹ and B² are each independently secondary C₃-C₈-alkyl or tertiary C₄-C₈₋alkyl, C₅-C₁₈-aryl or bis(C₅-C₁₈-aryl), or B¹ and B² together are a divalent radical which is selected from the group of 1,2-phenylene, 1,3-phenylene, 1,2-cyclohexyl, 1,1'-ferrocenyl, 1,2-ferrocenyl, 2,2'-(1,1'-binaphthyl) and 1,1'-biphenyl, and the radicals mentioned may optionally be mono- or polysubstituted by cyano, chlorine, fluorine, C₁-C₁₂-alkyl, C₁-C₁₂-haloalkyl, C₁-C₁₂-alkoxy, C₁-C₁₂-haloalkoxy, di(C₁-C₈-alkyl)amino or tri(C₁-C₈₋alkyl)siloxyl.

10. Process according to at least one of Claims 1 to 9, **characterized in that** the reaction is effected in the presence of compounds of the formula (I) in which B³ is secondary C₃-C₈-alkyl or tertiary C₄-C₈-alkyl, C₅-C₁₈-aryl, C₆-C₁₉₋arylalkyl or a radical having a total of from 2 to 40 carbon atoms and the valency n.

11. Process according to at least one of Claims 1 to 10, **characterized in that** the reaction is effected in the presence of compounds of the formula (I) in which X is chloride, bromide or iodide, trifluoromethanesulphonate, trifluoroacetate, methanesulphonate, benzenesulphonate, cyanide, optionally fluorinated acetylacetonate, hexafluorophosphate or tetrafluoroborate.

12. Process according to at least one of Claims 1 to 11 **characterized in that** the reaction is effected in the presence of compounds of the formula (I) in which n is 1 or 2 and m is 1 or 2.

13. Process according to at least one of Claims 1 to 12, **characterized in that** the reaction is effected in the presence of compounds of the formula (I) which contain the following phosphorus compounds as ligands: bis(2-dicyclohexylphosphino)-2'-(N,N-dimethylamino)biphenyl, 2-(dicyclohexylphosphino)biphenyl, 2-(dicyclohexylphosphino)-2'-methylbiphenyl, 2-(di-tert-butylphosphino)biphenyl, 2-(bisdiphenylphosphino)-binaphthyl, 1,1'-biphenyl-2-yl dicyclohexyl phosphonite, 1,1'-biphenyl-2-yl di-tert-butyl phosphonite, 3-[(diisopropylphosphino)oxy]phenyl diisopropyl phosphonite, 3-[(di-tert-butylphosphino)oxy]phenyl di-tert-butyl phosphonite, 3-[(diphenylphosphino)oxy]phenyl diphenyl phosphonite, 3-[(dicyclohexylphosphino)oxy]phenyl dicyclohexyl phosphonite, 1,1'-binaphthyl-2,2'-diyl isopropyl phosphite, tris(2,4-di-tert-butylphenyl) phosphite and 2,4,8,10-tetra-tert-butyl-6-phenoxy-12H-dibenzo[d,g][1,3,2]-dioxaphosphocine.

14. Process according to at least one of claims 1 to 13, **characterized in that** the reaction is effected in the presence of compounds of the formula (I) selected from [(µ-Br)2{2-(di-tert-butylphosphino)biphenyl}₂Cu₂], [(µ-Br)₂{2-(di-tert-butylphosphino)biphenyl}₂Cu₂], [(µ-trifluoromethanesulphonato)₂ {2-(di-tert-butylphosphino)-biphenyl}₂Cu₂], [(µ-Br)₂{2-(dicyclohexylphosphino)biphenyl}₂Cu₂], [(µ-trifluoromethanesulphonato)₂{2-(dicyclohexylphosphino)biphenyl}₂Cu₂] and [(µ-Br)₂{1,1'-binaphthyl-2,2'-diyl isopropyl phosphite)₂Cu₂].

15. Compounds of the formula (I) 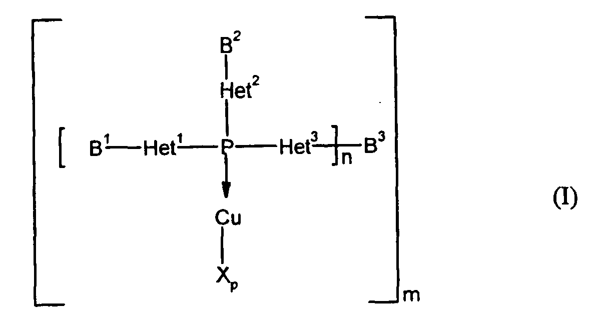 in which
Het¹, Het² and Het³ are each independently absent, or are oxygen or NR¹ where R¹ is C₁-C₈-alkyl, C₅-C₁₈-aryl or C₆-C₁₉-arylalkyl and
B¹ and B² are each independently C₁-C₈-alkyl, C₅-C₁₈-aryl or C₆-C₁₉-arylalkyl, or the B¹ and B² radicals together may be a divalent radical having a total of 2 to 40 carbon atoms and
X is halide, (C₁-C₈-haloalkyl)carboxylate, (C₁-C₈-alkyl)carboxylate, (C₁-C₈-haloalkyl)sulphonate, (C₅-C₁₈-aryl)sulpbonate, cyanide, optionally fluorinated acetylacetonate, nitrate, oxinate, phosphate, carbonate, hexafluorophosphate, tetraphenylborate, tetrakis-(pentafluorophenyl)borate or tetrafluoroborate, and
p is 0, 1 or 2 and
n is 1, 2 or 3 and
m is 1, 2 , 3, 4, 5 or 6,
**characterized in that** they contain the following phosphorus compounds as ligands: bis(2-dicyclohexylphosphino)-2'-(N,N-dimethylamino)biphenyl, 2-(dicyclohexylphosphino)biphenyl, 2-(dicyclohexylphosphino)-2'-methylbiphenyl, 2-(di-tert-butylphosphino)biphenyl, 2-(bisdiphenylphosphino)-binaphthyl, 1,1'-biphenyl-2-yl dicyclohexyl phosphonite, 1,1'-biphenyl-2-yl di-tert-butyl phosphonite, 3-[(diisopropylphosphino)oxy]phenyl diisopropyl phosphonite, 3-[(di-tert-butylphosphino)oxy]phenyl di-tert-butyl phosphonite, 3-[(diphenylphosphino)oxy]phenyl diphenyl phosphonite, 3-[(dicyclohexylphosphino)oxy]phenyl dicyclohexyl phosphonite, 1,1'-binaphthyl-2,2'-diyl isopropyl phosphite, tris (2,4-di-tert-butylphenyl) phosphite and 2,4,8,10-tetra-tert-butyl-6-phenoxy-12H-dibenzo[d,g] [1,3,2]dioxaphosphocine.

16. [(µ-Br)₂{2-(di-tert-butylphosphino)biphenyl}₂Cu₂], [(µ-trifluoromethanesulphonato)₂ {2-(di-tert-butylphosphino)-biphenyl}₂Cu₂], [(µ-Br)₂{2-(dicyclohexylphosphino)biphenyl}₂Cu₂], [(µ-trifluoromethanesulphonato)₂{2-(dicyclohexylphosphino)biphenyl}₂Cu₂] and [(µ-Br)₂{1,1'-binaphthyl-2,2'-diyl isopropyl phosphite)₂Cu₂].

17. Use of compounds according to at least one of Claims 15 and 16 for forming carbon-nitrogen, carbon-oxygen and carbon-sulphur bonds, and also for preparing arylalkines.

18. Catalysts comprising compounds according to at least one of Claims 15 and 16.

## Revendications

1. Procédé de préparation de composés de formule (IV),
Ar-(F-R²)ₙ (IV)
dans laquelle
n vaut 1, 2 ou 3,
Ar représente un radical aromatique substitué ou non substitué,
F représente un oxygène, un soufre, un NR³, un NR³CO ou un éthynediyle, où R³ représente un hydrogène, un alkyle en C₁-C₁₂, un aryle C₅-C₁₈ ou un arylalkyle en C₆-C₁₉, et
R² représente un Ar, un alkyle en C₁-C₁₂, un halogénoalkyle en C₁-C₁₂, un alcényle en C₂-C₁₂ ou un arylalkyle en C₆-C₁₉,
**caractérisé en ce que** des composés de formule (V)
Ar-Z (V)
Ar présente la signification mentionnée ci-dessus, et
Z représente un chlore, un brome, un iode, un sel de diazonium ou un sulfonate,
sont mis à réagir avec des composés de formule (VI)
H-F-R² (VI)
dans laquelle
F et R² présentent la signification mentionnée ci-dessus, et
où la réaction est réalisée en présence d'une base et de composés de formule (I), 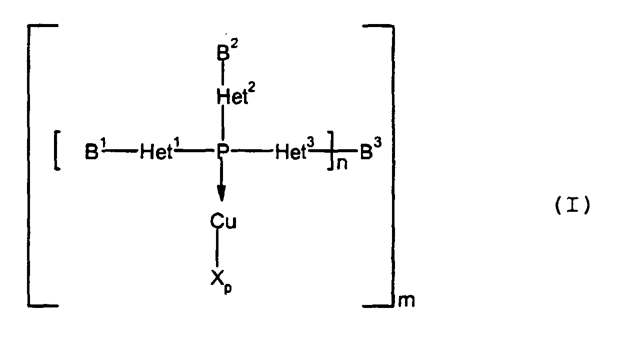 dans laquelle
Het¹, Het² et Het³, chacun indépendamment les uns des autres, sont absents ou représentent un oxygène ou un NR¹, dans lequel R¹ représente un alkyle en C₁-C₈, un aryle en C₅-C₁₈ ou un arylalkyle en C₆-C₁₉, et
B¹ et B², chacun indépendamment l' un de l'autre, représentent un alkyle en C₁-C₈, un aryle en C₅-C₁₈ ou un arylalkyle en C₆-C₁₉, ou les radicaux B¹ et B² peuvent représenter conjointement un radical bivalent ayant au total de 2 à 40 atomes de carbone, et
B³ représente un alkyle en C₁-C₈, un aryle en C₅-C₁₈ ou un arylalkyle en C₆-C₁₉ ou un radical ayant au total de 2 à 40 atomes de carbone et de valence n,
X représente un halogénure, un carboxylate d'halogénoalkyle en C₁-C₈, un carboxylate d'alkyle en C₁-C₈, un sulfonate d'halogénoalkyle en C₁-C₈, un sulfonate d'aryle en C₅-C₁₈, un cyanure, un acétylacétonate éventuellement fluoré, un nitrate, un oxinate, un phosphate, un carbonate, un hexafluorophosphate, un tétraphénylborate, un tétrakis(pentafluorophényl)borate ou un tétrafluoroborate, et
p vaut 0, 1 ou 2,
n vaut 1, 2 ou 3, et
m vaut 1, 2, 3, 4, 5 ou 6.

2. Procédé selon la revendication 1, **caractérisé en ce que** les composés de formule (I) sont utilisés en tant que composés isolés ou générés in situ.

3. Procédé selon au moins l'une des revendications 1 et 2, **caractérisé en ce que** Ar représente les radicaux aromatiques carbocycliques ayant de 6 à 24 atomes de carbone du squelette ou des radicaux hétéroraromatiques ayant de 5 à 24 atomes du squelette, dans lesquels zéro, un, deux ou trois atomes du squelette par cycle, mais au moins un atome du squelette dans l'ensemble de la molécule sont des hétéroatomes, qui sont choisis parmi le groupe constitué d'un azote, d'un soufre ou d'un oxygène, et où les radicaux aromatiques carbocycliques ou les radicaux hétéroaromatiques peuvent être substitués par jusqu'à cinq substituants identiques ou différents par cycle, qui sont choisis parmi le groupe constitué d'un hydroxy, d'un chlore, d'un fluor, d'un nitro, d'un cyano, d'un formyle libre ou protégé, d'un alkyle en C₁-C₁₂, d'un aryle en C₅-C₁₄, d'un arylalkyle en C₆-C₁₅, d'un -PO-[(C₁-C₈)-alkyle]₂, d'un -PO-[(C₅₋C₁₄)-aryle]₂, d'un -PO-[(C₁-C₈)-alkyl) (C₅-C₁₄) aryle], d'un tri(C₁-C₈-alkyl)siloxyle ou de radicaux de formules (VIIa-f)
A-B-D-E (VIIa) A-E (VIIb)
A-SO₂-E (VIIc) A-B-SO₂R⁴ (VIId)
A-SO₃W (VIIe) A-COW (VIIf)
dans lesquelles, indépendamment les uns des autres,
A est absent ou représente un radical alkylène en C₁-C₈,
B est absent ou représente un oxygène, un soufre ou un NR⁴,
dans lequel R⁴ représente un hydrogène, un alkyle en C₁-C₈, un arylalkyle en C₆-C₁₅ ou un aryle en C₅₋C₁₄,
D représente un groupe carbonyle,
E représente un R⁵, OR⁵, un NHR⁶ ou un N(R⁶)₂,
dans lesquel R⁵ représente un alkyle en C₁-C₈, un arylalkyle en C₆-C₁₅, un halogénoalkyle en C₁-C₈ ou un aryle en C₅-C₁₄, et
R⁶ représente chacun indépendamment un alkyle en C₁-C₈, arylalkyle en C₆-C₁₅ ou un aryle en C₅-C₁₄, ou N(R⁶)₂ représente conjointement un radical amino cyclique, et
W représente un OH, un NH₂ ou un OM dans lequel M peut représenter un ion de métal alcalin, un demi-équivalent d'un ion de métal alcalino-terreux, un ion ammonium ou un ion ammonium organique.

4. Procédé selon au moins l'une des revendications 1 à 3, **caractérisé en ce que** R² représente un Ar ou un alkyle en C₁-C₁₂.

5. Procédé selon au moins l'une des revendications 1 à 4 **caractérisé en ce que** les composés de formule (I) sont utilisés en quantités de 0,02 en moles à 10% en moles, par rapport au composés utilisés de formule (IV).

6. Procédé selon au moins l'une des revendications 1 à 5, **caractérisé en ce que** l'on utilise, en tant que base, des carbonates, des hydrogénocarbonates, des alcoolates, des phosphates, des fluorures et/ou des hydroxydes de métaux alcalins et/ou alcalino-terreux.

7. Procédé selon au moins l'une des revendications 1 à 6, **caractérisé en ce que** les bases utilisées sont pré-traitées par broyage et/ou séchage.

8. Procédé selon au moins l'une des revendications 1 à 7, **caractérisé en ce que** la réaction est réalisée en présence de composés de formule (I) dans laquelle Het¹, Het² et Het³, chacun indépendamment les uns des autres, représentent un oxygène ou sont absents.

9. Procédé selon au moins l'une des revendications 1 à 8, **caractérisé en ce que** la réaction est réalisée en présence de composés de formule (I) dans laquelle B¹ et B², chacun indépendamment l'un de l'autre, représentent un alkyle en C₃-C₈ ou un C₄-C₈ secondaire ou tertiaire, un aryle en C₅-C₁₈ ou un bis (C₅-C₁₈-aryle), ou B¹ et B² représentent conjointement un radical bivalent qui est choisi parmi le groupe constitué d'un 1,2-phénylène, d'un 1,3-phénylène, d'un 1,2-cyclohexyle, d'un 1,1'-ferrocényle, d'un 1,2-ferrocényle, d'un 2,2'-(1,1'-binaphtyle) et d'un 1,1'-biphényle, où les radicaux mentionnés peuvent être éventuellement mono- ou poly-substitués par un cyano, un chlore, un fluore, un alkyle en C₁-C₁₂, un halogénoalkyle en C₁-C₁₂, un alcoxy en C₁-C₁₂, un halogénoalcoxy en C₁-C₁₂, un di (C₁-C₈-alkyl) amino ou un tri (C₁-C₈₋alkyl)siloxyle.

10. Procédé selon au moins l'une des revendications 1 à 9, **caractérisé en ce que** la réaction est réalisée en présence de composés de formule (I) dans laquelle B³ représente un alkyle en C₃-C₈ ou en C₄-C₈ secondaire ou tertiaire, un aryle en C₅-C₁₈, un arylalkyle en C₆-C₁₉ ou un radical ayant au total de 2 à 40 atomes de carbone et de valence n.

11. Procédé selon au moins l'une des revendications 1 à 10, **caractérisé en ce que** la réaction est réalisée en présence de composés de formule (I) dans laquelle X représente un chlorure, un bromure ou un iodure, un trifluorométhanesulfonate, un trifluoroacétate, un méthanesulfonate, un benzènesulfonate, un cyanure, un acétylacétonate éventuellement fluoré, un hexafluorophosphate ou un tétrafluoroborate.

12. Procédé selon au moins l'une des revendications 1 à 11, **caractérisé en ce que** la réaction est réalisée en présence de composés de formule (I) dans laquelle n vaut 1 ou 2 et m vaut 1 ou 2.

13. Procédé selon au moins l'une des revendications 1 à 12 **caractérisé en ce que** la réaction est réalisée en présence de composés de formule (I) qui comprennent des composés phosphorés suivants en tant que ligands : un bis(2-dicyclohexylphosphino)-2'-(N,N-diméthylamino)biphényle, un 2-(dicyclohexylphosphino)biphényle, un 2-(dicyclohexylphosphino)-2'-méthylbiphényle, un 2-(di-tert-butylphosphino)-biphényle, un 2-(bisdiphénylphosphino)binaphtyle, un 1,1'-biphényl-2-yl-dicyclohexylphosphonite, un 1,1'-biphényl-2-yl-di-tert-butylphosphonite, un 3-[(diisopropylphosphino)oxy]phényldiisopropylphosphonite, un 3-[(di-tert-butylphosphino)oxy]phényl di-tert-butylphosphonite, un 3-[(diphénylphosphino)oxy]phényl-diphénylphosphonite, un 3-[(dicyclohexylphosphino)oxy]phényldicyclohexylphosphonite, un 1,1'-binaphthyl-2,2'-diyl-isopropylphosphite, un tris(2,4-di-tert-butylphényl)phosphite et une 2,4,8,10-tétra-tert-butyl-6-phénoxy-12H-dibenzo[d,g][1,3,2]-dioxaphosphocine.

14. Procédé selon au mois l'une des revendications 1 à 13, **caractérisé en ce que** la réaction est réalisée en présence de composés de formule (I) choisis parmi le [(µ-Br)₂{2-(di-tert-butylphosphino)-biphényl}₂Cu₂], le [(µ-trifluorométhanesulfonato)₂₋{2-(di-tert-butylphosphino)biphényl}₂Cu₂], le [(µ-Br)₂{2-(dicyclohexylphosphino)biphényl}₂Cu₂], le [(µ-trifluorométhanesulfonato)₂{2-(dicyclohexylphosphino)biphényl}₂Cu₂] et le [(µ-Br)₂{1,1'-binaphtyl-2,2'-diyl-isopropylphosphite)₂Cu₂].

15. Composés de formule (I). 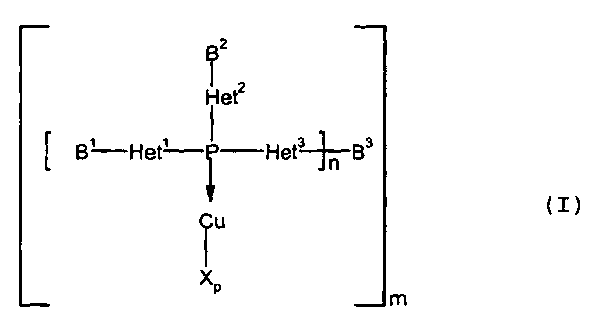 dans laquelle
Het1, Het2 et Het3, chacun indépendamment les uns des autres, sont absents ou représentent un oxyène ou un NR¹, dans lequel R¹ représente un alkyle en C₁-C₈, un aryle en C₅-C₁₈ ou un arylalkyle en C₆-C₁₉, B¹ et B², chacun indépendamment l'un de l'autre, représentent un alkyle en C₁-C₈, un aryle en C₅-C₁₈ ou un arylalkyle en C₆-C₁₉, ou les radicaux B¹ et B² peuvent représenter conjointement un radical bivalent ayant au total de 2 à 40 atomes de carbone, et
X représente un halogénure, un carboxylate d'halogénoalkyle en C₁-C₈, un carboxylate d'alkyle en C₁-C₈, un sulfonate d'halogénoalkyle en C₁-C₈, un sulfonate d'aryle en C₅-C₁₈, un cyanure, un acétylacétonate évenutellement fluoré, un nitrate, un oxinate, un phosphate, un carbonate, un hexafluorophosphate, un tétraphénylborate, un tétrakis(pentafluorophényl)borate ou un tétrafluoroborate,
p vaut 0, 1 ou 2,
n vaut 1, 2 ou 3, et
m vaut 1, 2, 3, 4, 5 ou 6,
**caractérisés en ce qu'**ils comprennent des composés phosphorés suivants en tant que ligands :
un bis(2-dicyclohexylphosphino)-2'-(N,N-diméthyamino)biphényle, un 2-(dicyclohexylphosphino)biphényle, un 2-(dicyclohexylphosphino)-2'-méthylbiphényle, un 2-(di-tert-butylphosphino)-biphényle, un 2-(bisdiphénylphosphino)binaphtyle, un 1,1'-biphényl-2-yl-dicyclohexylphosphonite, un 1,1'-biphényl-2-yl-ditert-butylphosphonite, un 3-[(diisopropylphosphino)oxy]phényldiisopropylphosphonite, un 3-[(di-tert-butylphosphino)oxylphényl di-tert-butylphosphonite, un 3-[(diphénylphosphino)oxy]phényl-diphénylphosphonite, un 3-[(dicyclohexylphosphino)oxy]phényldicyclohexylphosphonite, un 1,1'-binaphthyl-2,2'-diyl-isopropylphosphite, un tris(2,4-di-tert-butylphényl)phosphite et une 2,4,8,10-tétra-tert-butyl-6-phénoxy-12H-dibenzo[d,g][1,3,2]-dioxaphosphocine.

16. [(µ-Br)₂(2-(di-tert-butylphosphino)biphényl}₂Cu₂], [(µ-trifluorométhanesulfonato)₂{2-(di-tert-butylphosphino)biphényl}₂Cu₂], [(µ-Br)₂{2-(dicyclohexylphosphino)biphényl}₂Cu₂], [(µ-trifluorométhanesulfonato)₂{2-(dicyclohexylphosphino)biphényl}₂Cu₂] et [(µ-Br)₂(1,1'-binaphtyl-2,2'-diyl-isopropylphosphite)₂Cu₂].

17. Utilisation de composés selon au moins l'une des revendications 15 et 16 pour former des liaisons carbone-azote, carbone-oxygène et carbone-soufre ainsi que pour préparer des arylalcynes.

18. Catalyseurs comprenant des composés selon au moins l'une des revendications 15 et 16.
